# EUROPEAN PATENT APPLICATION

(11) **EP 4 696 311 A1**
(43) Date of publication of application: **18.02.2026**
(21) Application number: 24788294.7
(22) Date of filing: 12.04.2024
(51) Int. Cl.: A61K 35/16, A61K 35/19

(54) **PLASMA ENRICHED WITH PLATELETS AND PLASMA MOLECULES**

(30) Priority: 14.04.2023 ES 202330300
(71) Applicant: Santxa Research S.L.U., 01008 Vitoria-Gasteiz, Àlava (ES); Universidad del País Vasco/Euskal Herriko Unibertsitatea, 48940 Leioa - Vizcaya (ES)
(72) Inventor: SÁNCHEZ ÁLVAREZ, Mikel, 01008 Vitoria-Gasteiz (ES); SÁNCHEZ ARIZMENDIARRIETA, Pello, 01008 Vitoria-Gasteiz (ES); DELGADO SAN VICENTE, Diego, 01008 Vitoria-Gasteiz (ES); BEITIA SAN VICENTE, Maider, 01008 Vitoria-Gasteiz (ES); MERCADER RUIZ, Jon, 01008 Vitoria-Gasteiz (ES); BASABE DESMONTS, Lourdes, 48940 Leioa, Vizcaya (ES); BENITO LÓPEZ, Fernando, 48940 Leioa, Vizcaya (ES)
(74) Representative: ABG Intellectual Property Law, S.L.
(86) International application number: PCT/ES2024/070220
(87) International publication number: WO 2024/213815

(57) **Abstract**

The invention relates to a method for producing a plasma enriched in platelets and plasma biomolecules, and to the produced plasma itself. The method of the invention comprises centrifuging, evaporating and adjusting the pH of a blood sample.

## Description

### FIELD OF THE INVENTION

The present invention belongs to the field of biotechnology and relates to a method for obtaining a plasma enriched in platelets and plasma molecules and to the plasma obtained by means of said method.

### BACKGROUND OF THE INVENTION

In recent years, advanced biological therapies and regenerative medicine have become a promising alternative to conventional treatments. Platelet-rich plasma (PRP) is one of the technologies that has become most widespread and established in many fields of medicine. This is evidenced by the economic impact on the global market, where the technology was assessed at $45 million in 2009 and $120 million in 2016.

PRP is a group of blood products in which platelets are found at higher concentrations than in blood. Once the PRP is activated, plasma fibrinogen is polymerized into a three-dimensional transient fibrin scaffold, trapping various growth factors, microparticles and other biomolecules released by platelet and plasma degranulation. The growth factors and biomolecules sequestered in the fibrin scaffold are released gradually and in a sustained manner as the scaffold undergoes fibrinolysis, so PRP is very suitable for improving and accelerating the natural tissue repair process and ultimately to reduce recovery times.

In particular, the released growth factors trigger biological processes intended to repair damaged tissue, for example, angiogenesis, chemotaxis, cell migration or proliferation by means of cell membrane signaling. Some types of growth factors circulate in plasma, for example, insulin-like growth factor (IGF), which promotes wound healing, bone formation, striated muscle myogenesis and keratinocyte migration and has proven to be particularly useful in musculoskeletal regenerative therapy, such as in articular cartilage repair, or against osteoporosis, age-associated wasting, muscular dystrophy or osteoarthritis, among others (Leon A Bach, Clin Biochem Rev Vol 25 155-160, August 2004).

Specific clinical practices in which PRP-based therapies have made their mark are orthopedics and sports medicine. This is evidenced by the increasing studies related to pathologies such as osteoarthritis, tendinopathies or ligament injuries. As a result, a large amount of PRP products have emerged on the market and, although these products are generally labeled as PRP, they have different properties, such as varying concentrations of platelets, plasma molecules, or the presence or absence of leukocytes. The device and method used in the preparation of PRP has a significant impact on said properties. Examples of commercially available systems for preparing PRP are Magellan (Arteriocyte Medtronic), ACP or Angel (Arthrex), PRGF-Endoret (BTI Biotechnology Institute), MTF (Cascade), Secquire (Pure PRP Emcyte), RegenKit (RegenLab), GPS (Zimmer Biomet), Ortho Pras (Proteal).

Despite the large number of PRP systems on the market, they are all based on the principle of centrifugation to concentrate platelets. This technique generates a concentration gradient based on the weight of blood components and allows isolating and concentrating platelets. As a result of the increase in platelet concentration, platelet growth factors stored in these platelets also increase. However, the centrifugation speed used in these methods cannot concentrate many non-platelet (extraplatelet) biomolecules found in plasma to the same extent as the platelets or platelet growth factors mentioned above. Obtaining said non-platelet molecules by centrifugation would involve very high centrifugation speeds which are not compatible with cell viability or with daily medical practice.

Although the PRPs obtained by the aforementioned methods of the prior art are achieving promising results, there is a constant need for the development of new methodologies that are capable of producing next generation PRP that allow for more effective medical therapies.

### SUMMARY OF THE INVENTION

The development of a new methodology for producing a completely natural plasma enriched in platelets and plasma molecules is shown herein. This allows a high level of biosafety and utilizes the biological properties of the biomolecules present in the plasma.

As shown in the examples of the application, the authors of the present invention have demonstrated that the plasma of the invention is superior to conventional plasmas in terms of the proportion of plasma proteins present therein and of its capacity to induce dermal fibroblast cell proliferation. In particular, the plasma obtained by means of the developed method surprisingly contains a higher concentration of the growth factor IGF-I compared to that of other plasmas of the state of the art.

The new method is very advantageous because it requires minimal preparation for use thereof by physicians.

Therefore, in a first aspect the invention relates to an *in vitro* method for producing a plasma enriched in platelets and plasma molecules which comprises:
i) obtaining a red blood cell-free plasma from an anticoagulated blood sample of a subject, wherein the plasma comprises platelets and plasma molecules;
ii) concentrating the platelets and plasma molecules present in the plasma obtained in step i) by means of evaporation;
iii) adjusting the pH of the concentrated plasma obtained in step ii) to a value of between 6.5 and 7.5, more preferably 7.4.

In a second aspect, the invention relates to a plasma enriched in platelets and plasma molecules obtained by means of the method of the first aspect of the invention.

In a third aspect, the invention relates to a plasma enriched in platelets and plasma molecules according to the second aspect of the invention for use in medicine.

In one embodiment, the invention relates to a plasma enriched in platelets and plasma molecules according to the second aspect of the invention for use in the treatment of injured tissues.

In one embodiment, the invention relates to a plasma enriched in platelets and plasma molecules according to the second aspect of the invention for use in the treatment of inflammatory pathologies.

In another aspect, the invention relates to the cosmetic use of the plasma enriched in platelets and plasma molecules according to the second aspect of the invention.

### BRIEF DESCRIPTION OF THE FIGURES

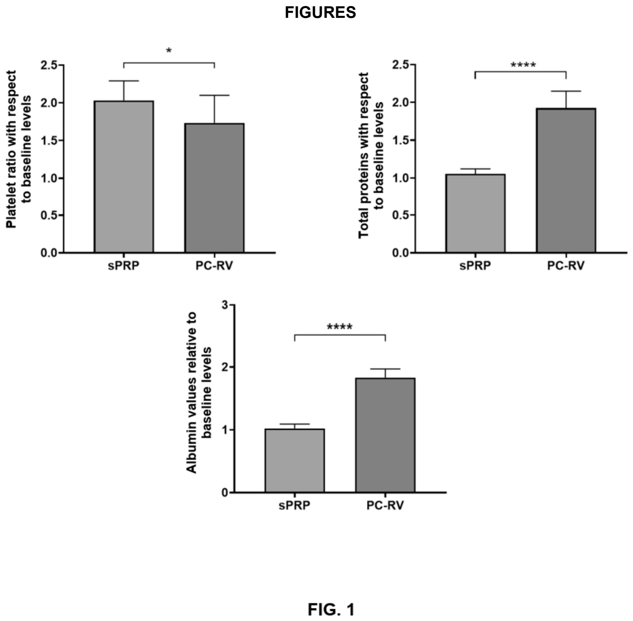

**Figure 1****.** Relative levels of platelets, total proteins and albumin in different plasma formulations. The statistical significance between the new plasma (PC-RV) and a conventional/standard commercially available PRP (sPRP) was calculated by means of the t-test (* p<0.05 **** p<0.0001).

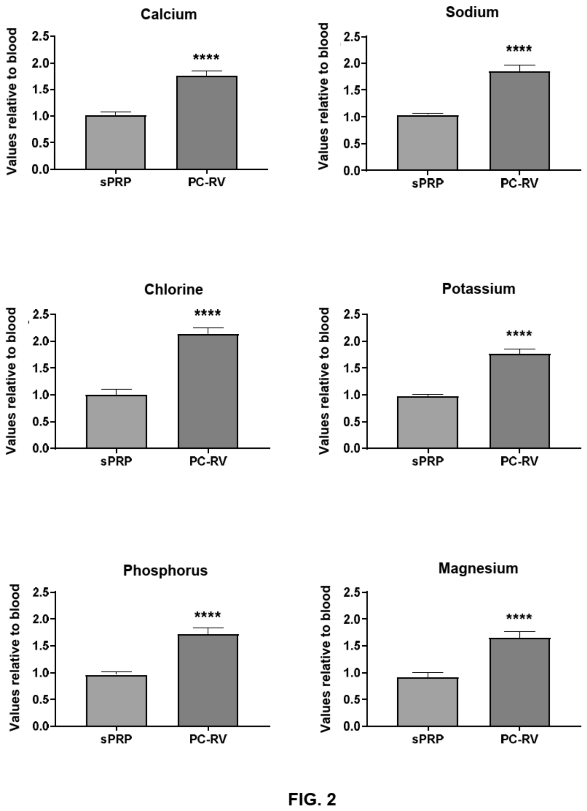

**Figure 2****.** Relative levels of ions in different plasma formulations. Plasmas obtained by means of the different systems were analyzed to determine their calcium, sodium, chlorine, potassium, phosphorus and magnesium contents. The statistical significance between the new plasmas and sPRP was calculated by means of the t-test (**** p<0.0001).

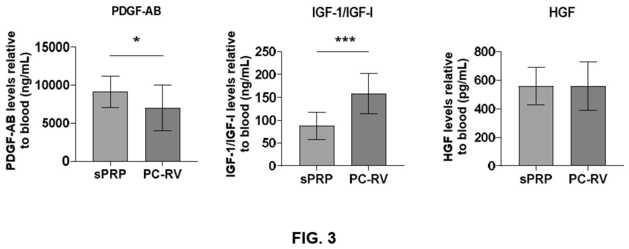

**Figure 3****.** Relative levels of growth factors in different plasma formulations. Plasmas obtained were analyzed to determine their PDGF, HGF and IGF-1 contents. The statistical significance between the new plasmas and sPRP was calculated by means of the t-test (* p<0.05; **** p<0.001).

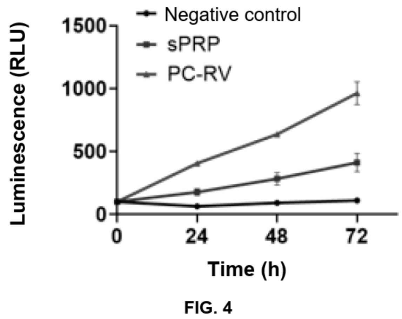

**Figure 4****.** Growth curve of dermal fibroblasts cultured with different sera. The cells were cultured with media supplemented with sera originating from different concentrated plasmas, sPRP or PC-RV, and viability was measured every 24 h for a total of 72 h.

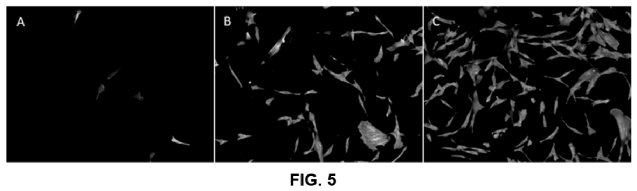

**Figure 5****.** Protein expression of Phalloidin (green) and Hoechst (blue) by means of immunofluorescence in dermal fibroblasts. The cells were cultured in media supplemented with sera from different plasma formulations, sPRP and PC-RV. After 72 h the cells were stained by means of immunofluorescence to observe the cell density at that time.

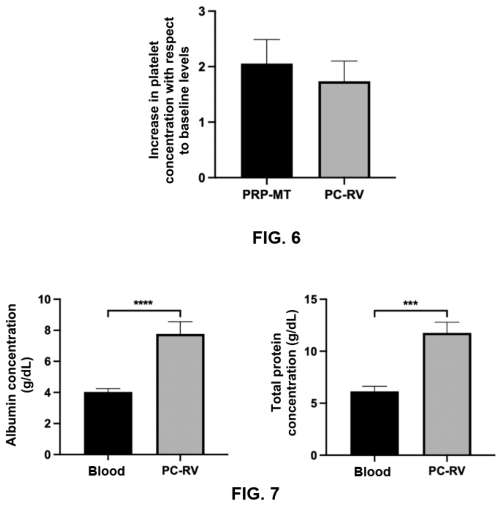

**Figure 6****.** Relative levels of platelets in PC-RV and PRP-MT formulations. Plasmas obtained by means of the different systems were analyzed to determine their platelet contents, which were relativized with respect to baseline levels in blood. The statistical significance between the two PRPs was calculated by means of Student's t-test.
**Figure 7****.** Relative levels of total proteins and albumin of PC-RV relative to blood. Plasmas obtained were analyzed to determine their protein and albumin contents, which were relativized with respect to baseline levels in blood. The statistical significance for albumin and total protein concentration between PC-RV and blood was calculated by means of Student's t- and Wilcoxon tests, respectively (*** p<0.001; **** p<0.0001).

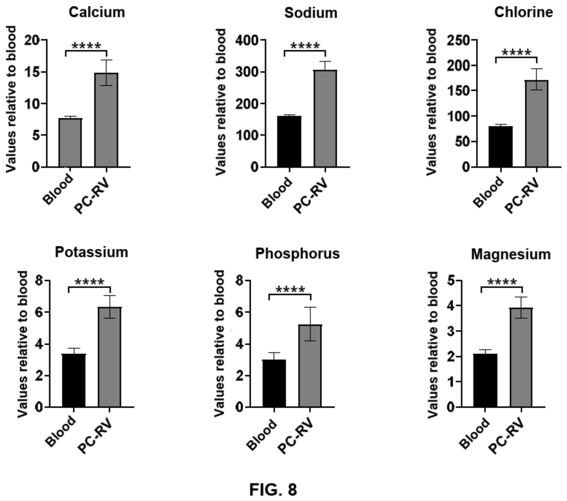

**Figure 8****.** Relative levels of PC-RV ions relative to blood. Plasmas obtained were analyzed to determine their calcium, sodium, chlorine, potassium, phosphorus and magnesium contents. The statistical significance between PC-RV and blood was calculated by means of Student's t-test (**** p<0.0001).

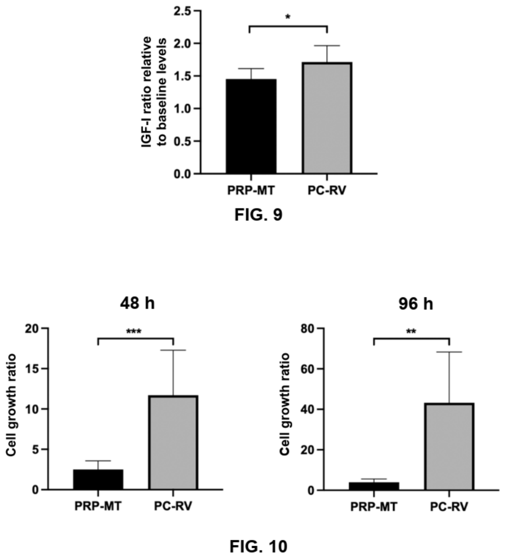

**Figure 9****.** Relative levels of plasma growth factors in different plasma formulations. Plasmas obtained by means of different PRP-MT and PC-RV systems were analyzed to determine their IGF-1 contents. Statistical significance was calculated by means of Student's t-test (* p<0.05).
**Figure 10****.** Growth curve of dermal fibroblasts cultured with different sera. The cells were cultured with media supplemented with sera originating from different concentrated plasmas and viability was measured every 0, 48 and 96 h. The statistical significance between PRP-MT and PC-RV for each time was calculated by means of Student's t-test with Welch's correction (** p<0.01; *** p<0.001).

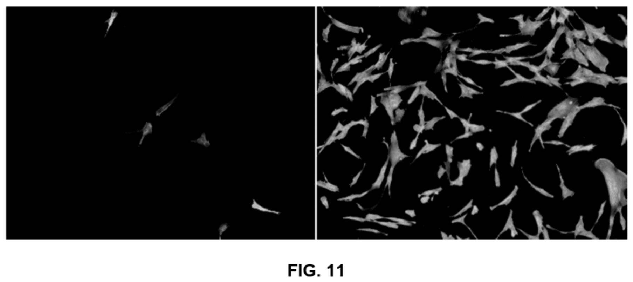

**Figure 11****.** Protein expression of Phalloidin (green) and Hoechst (blue) by means of immunofluorescence in dermal fibroblasts. The cells were cultured in medium supplemented with PC-RV. The cells were stained by means of immunofluorescence at the beginning and after 72 h to observe the increase in cell density at that time.

### DETAILED DESCRIPTION OF THE INVENTION

Unless otherwise indicated, all the terms as used herein will be understood in their common meaning as known in the art. Other more specific definitions for certain terms as used herein will be defined below in this section and are intended to be applied uniformly throughout the specification and claims unless a definition expressly stated otherwise provides a broader definition.

### Method of the invention

In a first aspect, the invention relates to an *in vitro* method for producing a plasma enriched in platelets and plasma molecules which comprises:
i) obtaining a red blood cell-free plasma from an anticoagulated blood sample of a subject, wherein the plasma comprises platelets and plasma molecules;
ii) concentrating the platelets and plasma molecules present in the plasma obtained in step i) by means of evaporation;
iii) adjusting the pH of the concentrated plasma obtained in ii) to a value of between 6.5 and 7.5, more preferably 7.4.

The term *"in vitro",* as used herein, refers to the fact that the method is not carried out in the body of a human or animal subject, but in cells or fluids isolated from said subject or in a test tube.

The term "subject", as used herein, refers to any animal with blood circulation, preferably a mammal, more preferably a primate, and even more preferably, a human being, male or female, of any age or race. Furthermore, the subject may be a healthy subject or a subject diagnosed with any pathology or disease.

The term "plasma" or the expression "blood plasma" in the present invention refers to the non-cellular liquid portion of human or animal blood before coagulation. It is composed of 90% water, 7% protein and the remaining 3% fat, glucose, vitamins, hormones, oxygen, carbon dioxide and nitrogen, in addition to metabolic waste products such as uric acid. It is the main component of blood, accounting for about 55% of the total blood volume, while the remaining 45% corresponds to formed elements. The viscosity of blood plasma is 1.5 times that of water. The main plasma proteins are the following: albumin, which is also involved in lipid transport; globulins, which are mainly related to the body's defense mechanisms (for example, immunoglobulins) or to iron transport (transferrin); fibrinogen, a protein that is essential for blood coagulation; prothrombin, a protein which is involved in the coagulation process; and low- and high-density lipoproteins (LDL and HDL, respectively) which are involved in cholesterol transport. The set of plasma proteins, also called "plasma factors", performs the functions, among others, of:
- maintaining plasma volume and blood volume,
- protecting and maintaining the stability of blood pH,
- being involved in blood viscosity, and therefore contributing minimally to peripheral vascular resistance and vascular pressure (blood pressure), and
- intervening in the concentration of electrochemical equilibrium ions (called Donnan effect).

The term "platelets", as used herein, refers to small (2-3 micrometers in diameter), oval, nucleus-free cell units generated under normal conditions in the bone marrow from the cytoplasmic fragmentation of megakaryocytes. Platelets circulate in the blood of all mammals and are involved in hemostasis, being involved in the formation of blood clots. Platelets release a large number of growth factors and/or cytokines, including platelet-derived growth factor (PDGF, also abbreviated as PDGFAA), a potent chemotactic agent, and transforming growth factor beta (TGF-β1), which stimulates the formation of extracellular matrix. Depending on certain conditions, platelets also synthesize many other molecules, as discussed above, which are essential to the inflammatory process, and to the tissue repair and regenerative process. These growth factors or molecules have been shown to play an important role in the regeneration and repair of connective tissue. Other proteins and growth factors produced by platelets and associated inflammatory, reparative and regenerative processes include basic fibroblast growth factor (BFGF), insulin-like growth factor 1 (IGF-1), insulin-like growth factor 2 (IGF-2), epithelial growth factor (EGF), hepatocyte growth factor (HGF), vascular endothelial growth factor (VEGF), interleukin 8, keratinocyte growth factor, connective tissue growth factor. Local application of all these growth factors, molecules and proteins produced by platelets is involved in and accelerates the healing process of different injuries. Platelets also release large amounts of thrombin and adenosine diphosphate ADP, calcium, thromboxane A2, PF4, PAI-1, fibrinogen, fibronectin, thrombomodulin, FV, FXIII, RANTES, thrombospondin, WWF PF-3, serotonin, hydrolytic enzymes, etc.

The term "plasma molecules," "plasma biomolecules" or "non-platelet biomolecules", as used herein, refers to biomolecules not stored or not exclusively stored in platelets.

Preferably, "plasma molecules" refers to growth factors. However, the term "plasma molecules" may also include ions and proteins other than growth factors. Preferably, the term "plasma molecules" refers to biomolecules that cannot be concentrated by means of centrifugation techniques without damaging the platelets and/or the biomolecules themselves after said centrifugation. Concentration refers to an increase in total protein concentration as defined below for the present invention. In this regard, the concentration of platelets and plasma molecules present in the anticoagulated blood is similar to the concentration of platelets and plasma molecules present in the red blood cell-free plasma obtained after the centrifugation process before concentration by evaporation.

According to the invention, "plasma protein" is understood to mean any protein, and more particularly any protein of industrial or therapeutic interest, contained in blood plasma. Blood plasma proteins include albumin, alpha/macroglobulin, antichymotrypsin, antithrombin, antitrypsin, Apo A, Apo B, Apo C, Apo D, Apo E, Apo F, Apo G, beta XIIa, C1-inhibitor, C-reactive protein, C7, C1r, C1s, C2 C3, C4, C4bP, C5, C6, C1q, C8, C9, carboxypeptidase N, ceruloplasmin, factor B, factor D, factor H, factor I, factor IX, factor V, factor VII, factor VIIa, factor VIII, factor X, factor XI, factor XII, factor XIII, fibrinogen, fibronectin, haptoglobin, hemopexin, heparin cofactor II, histidine-rich GP, IgA, IgD, IgE, IgG, ITI, IgM, kinase II, HPM kininogen, lysozyme, PAI 2, PAI I, PCI, plasmin, plasmin inhibitor, plasminogen, prealbumin, prekallikrein, properdin, protease nexin INH, protein C, protein S, protein Z, prothrombin, serum amyloid protein (SAP), TFPI, thiol proteinase, thrombomodulin, tissue factor (TF), TPA, transcobalamin II, transcortin, transferrin, vitronectin, and Willebrand factor. Blood plasma proteins also include, among others, some hormones, including insulin-like growth factors, e.g. IGF-I.

Particularly, plasma proteins include coagulation proteins, i.e., plasma proteins involved in the chain of cascade reactions leading to the formation of a blood clot. Coagulation proteins include factor I (fibrinogen), factor II (prothrombin), factor V (proaccelerin), factor VII (proconvertin), factor VIII (anti-hemophilic factor A), factor IX (anti-hemophilic factor B), factor X (Stuart factor), factor XI (Rosenthal factor or PTA), factor XII (Hageman factor), factor XIII (fibrin-stabilizing factor or FSF), PK (prekallikrein), KHPM (high molecular weight kininogen), factor III (thromboplastin or tissue factor), heparin cofactor II (HCII), protein C (PC), thrombomodulin (TM), protein S (PS), Willebrand factor (Wf) and tissue factor pathway inhibitor (TFPI), or also tissue factors.

In some embodiments, the plasma protein consists of a coagulation protein with enzymatic activity. Coagulation proteins with enzymatic activity include activated forms of factor II (prothrombin), factor VII (proconvertin), factor IX (anti-hemophilic factor B), factor X (Stuart factor), factor XI (Rosenthal factor or PTA), factor XII (Hageman factor), factor XIII (fibrin-stabilizing factor or FSF) and PK (prekallikrein).

As used herein, "plasma enriched in plasma molecules" refers to plasma that has undergone a process that increases its concentration of non-platelet biomolecules. In a preferred form, it refers to plasma that has been subjected to a process which increases the concentration of its total proteins as defined below.

The term "blood", as used herein, refers to a tissue fluid flowing through the capillaries, veins and arteries of all vertebrates. Its characteristic red color is due to the presence of the red pigment contents of erythrocytes. It is a type of specialized connective tissue with a liquid colloidal matrix and a complex constitution. In fact, blood comprises a solid phase formed by cells (including leukocytes, erythrocytes and platelets) and a liquid phase, represented by plasma. Its main function is logistic distribution and systemic integration, the containment of which in vessels (vascular space) supports its distribution (blood circulation) throughout most of the body.

The term "erythrocytes" or "red blood cells" or "RBC", in the sense used herein, refers to blood corpuscles that will transport oxygen and carbon dioxide in the blood. Corpuscles are known to lack nuclei and organelles (only in mammals), so they cannot be strictly considered as cells. They contain some enzymatic pathways and their cytoplasm is almost entirely occupied by hemoglobin, a protein responsible for oxygen transport. Glycoproteins (CDs) that define the different blood groups and other cell identifiers are found in the plasma membrane of erythrocytes. Erythrocytes are disk-shaped, biconcave, depressed in the center; this increases the effective surface area of the membrane. Mature RBCs lack a nucleus, because it is expelled at the last stage of maturation in the bone marrow before entering the bloodstream.

The term "anticoagulated" is used herein to refer to blood that has been treated with an anticoagulant. In turn, the term "anticoagulant" in the sense used herein refers to an endogenous or exogenous substance that interferes with or inhibits blood coagulation. Non-limiting examples of anticoagulants are antithrombin III activators and heparin, low molecular weight ardeparin, certoparin, nadroparin, logiparin, parnaparin, reviparin and tedelparin, and recombinant antithrombin III, tissue factor (TF)-Factor Vila inhibitor such as TF mutants, anti-TF antibodies, factor VII inhibitors, thrombin inhibitors such as antistatin factor Xa inhibitors, TAP (tick anticoagulant peptide), DX9065, lefaxine, fondaparinux, terostatin, YM-75466 and ZK-807,834, antibodies against factors IXa and Xa, protein C pathway activators and selective thrombin inhibitors such as argatroban, bivalirudin, efegatran, H376/95, hirugen, inogatran, melagatran, napsagatran, UK-156406 and ximelagatran, and chemical compounds that capture calcium ions, such as ethylenediaminetetraacetic acid (EDTA), citrate (generally sodium citrate) and oxalate.

In a particular embodiment, the anticoagulant is sodium citrate.

In a particular embodiment, the anticoagulant, preferably sodium citrate, is comprised in a solution, preferably an aqueous solution. In a particular embodiment, the sodium citrate is comprised in a solution, preferably an aqueous solution, comprising citric acid.

In another embodiment, the anticoagulant, preferably sodium citrate, is present in a concentration of between 1% and 10%, between 2% and 8%, between 2% and 6%, between 3% and 5%, between 3% and 4%, more specifically at a concentration of 3.8% (w/v), w/v referring to the weight of the anticoagulant with respect to the volume of the solution in g/100 mL.

As used in the present invention, the term "evaporation" refers to a type of vaporization of a liquid in which the surface of the liquid transitions into a gaseous phase that is not saturated with the substance being evaporated.

In a first step i), the method of the invention comprises obtaining a red blood cell-free plasma from an anticoagulated blood sample of a subject. Said step comprises treating a blood sample of a subject with an anticoagulant, wherein the meaning of anticoagulant is the same as that provided above.

In a particular embodiment, red blood cell-free plasma is obtained by means of centrifuging the anticoagulated blood sample at a speed of at least 700 g, at least 750 g, at least 800 g, at least 850 g, at least 900 g, at least 950 g, at least 1000 g, at least 1050 g, at least 1100 g, at least 1150 g, at least 1200 g, particularly up to: 1500 g, 1400 g or 1300 g. In one embodiment, the red blood cell-free plasma is obtained by means of centrifuging the anticoagulated blood sample for at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes, at least 16 minutes, at least 17 minutes, at least 18 minutes, at least 19 minutes or at least 20 minutes, particularly up to: 45 minutes, 40 minutes, 35 minutes, 30 minutes or 25 minutes. In another embodiment, these speeds and times are combined. One skilled in the art will know how to combine and vary the conditions of centrifugation speed and centrifugation time by modifying them in a compensatory manner, that is, by increasing the centrifugation force and reducing the centrifugation time or alternatively by reducing the centrifugation speed and increasing the time to obtain red blood cell-free plasma.

In a particular embodiment, the red blood cell-free plasma is obtained by means of centrifuging an anticoagulated blood sample at a speed of between 700 and 1500 g, preferably between 1050 and 1400 g, more preferably at 1200 g.

In another particular embodiment, the red blood cell-free plasma is obtained by means of centrifuging an anticoagulated blood sample for between 5 and 15 minutes, preferably between 5 and 11 minutes, more preferably for 8 minutes.

In another particular embodiment, the red blood cell-free plasma is obtained by means of centrifuging an anticoagulated blood sample at a speed of between 700 and 1500 g, preferably between 1050 and 1400 g, more preferably at 1200 g, and for 5-15 minutes, preferably for 5-11 minutes, more preferably for 8 minutes.

In a preferred embodiment, the red blood cell-free plasma is obtained by means of centrifuging an anticoagulated blood sample at a speed of 1200 g for 8 minutes.

After the centrifugation process, different layers of blood components are obtained: a plasma layer in the top part (supernatant) comprising platelets and plasma molecules; a buffy coat in the center comprising white blood cells; and a bottom layer comprising red blood cells.

The red blood cell-free plasma is obtained by collecting the supernatant and the central layer. This can be done by any means known to the one skilled in the art, such as pipetting, for example.

The term "centrifugation" refers to a method whereby solids of different densities are separated from liquids through a rotational force, which applies a centrifugal force greater than the force of gravity to the mixture. Solids and particles of higher density are deposited. Examples of centrifugation include, without limitation, differential centrifugation, isopycnic centrifugation, zonal centrifugation or ultracentrifugation. In the context of the present invention, differential centrifugation is preferred.

Centrifuges used for preparing red blood cell-free plasma can be obtained from a variety of sources, including, for example, the PLACONTM centrifuge, which can be obtained from OCT USA Inc., Torrence.

The term "red blood cell-free" refers to the fact that the plasma does not contain or contains very low amounts of red blood cells. In another particular embodiment, the plasma obtained does not contain red blood cells or contains less than 5% by weight, less than 4% by weight, less than 3% by weight, less than 2% by weight, less than 1% by weight of red blood cells with respect to the total weight of the plasma.

The red blood cell-free plasma contains a plasma concentration similar to that of the baseline levels in blood. Therefore, in a particular embodiment, the red blood cell-free plasma obtained after centrifugation contains a platelet concentration of between 100,000 and 400,000 platelets per microliter of blood (pl/µl), more specifically between 150,000 and 350,000 platelets per microliter of blood (pl/µl).

In another particular embodiment, the method of the invention comprises, in step i), obtaining a red blood cell- and white blood cell-free plasma from an anticoagulated blood sample of a subject.

The term "leukocyte" or the expression "white blood cells", used interchangeably herein, refers to blood cells that are part of the cellular effectors of the immune system. They are cells with migratory capacity that use blood as a vehicle to access different parts of the body. Leukocytes are responsible for destroying infectious agents and infected cells. Based on the microscopic characteristics of their cytoplasm (staining) and nucleus (morphology), they are divided into:
- granulocytes and polymorphonuclear cells (neutrophils, basophils and eosinophils) and have a polymorphic nucleus and numerous granules in the cytoplasm, with differential staining according to cell types; and
- agranulocytes or monomorphonuclear cells (lymphocytes and monocytes): they lack granules in the cytoplasm and have a round nucleus.

The term "red blood cell- and white blood cell-free" refers to the fact that the plasma does not contain red cells and white cells or contains very low amounts of red blood cells and white blood cells. In a particular embodiment, the plasma obtained does not contains red blood cells or white blood cells or contains less than 5% by weight, less than 4% by weight, less than 3% by weight, less than 2% by weight, less than 1% by weight of each of red blood cells and white blood cells with respect to the total weight of the plasma.

The red blood cell- and white blood cell-free plasma contains a plasma concentration similar to that of the baseline levels in blood. Therefore, in a particular embodiment, the red blood cell- and white blood cell-free plasma obtained after centrifugation contains a platelet concentration of between 100,000 and 400,000 platelets per microliter of blood (pl/µl), more specifically between 150,000 and 350,000 platelets per microliter of blood (pl/µl).

The red blood cell- and white blood cell-free plasma is obtained by means of centrifuging in the same manner as for obtaining the red blood cell-free plasma, but in this case collecting only the top layer, i.e., not collecting the buffy coat or the bottom layer.

In a second step ii), the method of the invention comprises concentrating the platelets and plasma molecules present in the plasma obtained in the first step i) by means of evaporation.

Evaporation can be performed by means of i) increasing the temperature, ii) reducing the pressure, iii) or by means of the combination of both effects, i.e., increasing the temperature and reducing the pressure; optionally while centrifuging the plasma.

For concentration by means of increasing the temperature, various means known to one skilled in the art may be used, including, without limitation, water baths or other heat sources.

For concentration by means of decreasing pressure, the plasma can be introduced into a vacuum desiccator in the presence of a desiccant substance or agent, alternative methods known in the technical field would also be contemplated in the present invention.

Evaporation by means of a combination of increasing the temperature and reducing the pressure can be performed by evaporation in vacuum ovens having a temperature regulator as well as a vacuum connection, such as in a rotary evaporator or a centrifugal evaporator.

More preferably, evaporation by means of a combination of increasing the temperature and reducing the pressure is performed by rotary evaporation, i.e., using a rotary evaporator. Through this technique, evaporation takes place at reduced pressure, normally produced by a vacuum pump, and at moderate temperatures, thus achieving the removal of vapors by heating and simultaneous application of vacuum. Rotary evaporation is essentially a distillation at reduced pressure: a solution (in this case the plasma) in a round-bottom flask, in particular, is placed in a water bath of the rotary evaporator and the flask is rotated while the system is partially evacuated (by means of a water suctioning device or a vacuum pump). The reduced pressure in the apparatus causes certain components of the plasma, such as water, to boil at a lower temperature than normal, and the rotation of the flask increases the surface of the liquid and therefore the evaporation speed. The vapor of the evaporated substance condenses when it comes into contact with a water condenser and drips into a receiving flask. When the evaporated substance is removed, the concentrated plasma is left in the flask. One difference between distillation and rotary evaporation is that the distillate is often retained in distillation, while it is the residue that is retained in rotary evaporation.

A centrifugal evaporator is an evaporator used to concentrate solutions by providing centrifugal force. The centrifugal evaporator is based on centrifugation, temperature and vacuum. The solution to be evaporated (in this case the plasma) is placed in the centrifugal evaporator and the desired temperature and centrifugation speed are applied. The centrifugal force generated by the rotation of the centrifuge rotor creates a pressure gradient in the plasma in the tubes or vials, which means that the samples boil from top to bottom, helping to prevent excessive boiling that may cause shaking. A vacuum is then established. This removes air and reduces, in particular, the pressure in the device, and therefore the boiling temperature of certain plasma components, such as water. When the pressure is so low that the boiling temperature drops below the temperature of the container, the plasma begins to evaporate. The process continues until the plasma has evaporated to the desired volume.

In a preferred embodiment, evaporation is performed by rotary evaporation.

To that end, plasma is introduced into a container or flask to proceed with evaporation using the rotary evaporator. The temperature of the water-filled bath of the rotary evaporator is adjusted and the container or flask with the plasma is introduced therein, rotating it so that the sample is distributed over the entire surface thereof, achieving faster evaporation of the sample. Finally, to be able to proceed with evaporation, a vacuum is applied. The process continues until the sample is concentrated to the desired volume.

In a particular embodiment, during evaporation, preferably rotary evaporation, the plasma is subjected to a temperature of between 30°C and 45°C, between 33°C and 40°C, more preferably 37°C.

In another particular embodiment, during evaporation, preferably rotary evaporation, the plasma is subjected to an applied vacuum of between 250 and 1000 millibars (mbar), more preferably between 300 and 1000 mbar, more preferably between 400 and 1000 mbar, between 500 and 1000 mbar, between 600 and 1000 mbar, between 700 and 1000 mbar, between 800 and 1000 mbar, between 900 and between 1000 mbar, more preferably to vacuum of about 920 mbar.

The term "applied vacuum" refers to the reduction in pressure with respect to atmospheric pressure. Therefore, if the atmospheric pressure is 1000 mbar, an applied vacuum of 920 mbar would result in a pressure of 80 mbar.

In a particularly preferred embodiment, the temperature of the evaporation, preferably rotary evaporation, indicated above is combined with each of these rotary evaporation vacuum ranges.

In a particular embodiment, the evaporation, preferably rotary evaporation, is performed for at least 1 minute. In another embodiment, for a period of between 1 minute and 30 minutes, 1 minute and 25 minutes, 1 minute and 20 minutes, 1 minute and 15 minutes, 1 minute and 10 minutes, or 1 minute and 5 minutes. More particularly, it is performed for at least one minute and at most 30 minutes. Generally, after 3-5 minutes the sample will be concentrated to half of its initial volume, for example from 12 mL to 6 mL. The process time can be increased or reduced to obtain a smaller or larger final volume, with higher or lower concentration. In one embodiment, the volume of the plasma is reduced by between 10 and 90%, more particularly between 30 and 70%, even more particularly between 40 and 60%.

Once the evaporation process has ended, the concentrated plasma sample is collected.

The method of the invention comprises a third step iii) which comprises measuring and adjusting the pH of the concentrated plasma.

The concentrated plasma sample obtained in step (ii) is collected and the pH is measured. pH measurement is a routine task that will be performed using means well known to any person skilled in the art.

The pH of the concentrated plasma is then adjusted to a pH of between 6 and 7.6; between 6.5 and 7.5; between 7 and 7.4; more preferably to a pH of 7.4. In a particular embodiment, pH adjustment is performed by means of adding a 0.8 M hydrochloric acid buffer at a percentage of 2.3%. According to common general knowledge, any type of buffer can be used to acidify the plasma obtained. pH adjustment is necessary for the desired coagulation to occur as a result of platelet activation prior to the use of said plasma.

In a preferred embodiment, the method of the invention further comprises the step of activating the platelets obtained in the concentrated plasma obtained after adjusting the pH.

The expression "platelet activation" refers herein to the process whereby platelets present in plasma undergo aggregation to cause clot formation and promote hemostasis and healing by means of, among other processes, the secretion of growth factors or intercellular mediators from cytoplasmic granules. Platelets can be activated by a large number of substances with which they react within seconds. When exposed to these agonists, platelets exhibit different responses, which are closely associated *in vivo,* but which can be studied separately *in vitro*: adhesion and shape change, aggregation, secretion and expression of procoagulant activity.

Activated platelets change their shape to become more spherical and form pseudopods on their surface. In this way, they adopt a star shape. After activation, granule secretion occurs. Platelets contain alpha granules and dense granules. Activated platelets excrete the contents of these granules into their canalicular systems and into the surrounding blood or plasma. There are two types of granules: (i) dense granules (containing ADP or ATP, calcium and serotonin) and (ii) α-granules (containing platelet factor 4, transforming growth factor beta 1 (TGF beta 1), platelet-derived growth factor, fibronectin, B-thromboglobulin, vWF, fibrinogen, and coagulation factors (factors V and VIII), among others).

In a particular embodiment, the platelets are activated by a substance selected from calcium chloride, thrombin and collagen. In another embodiment, the platelets are activated mechanically by means of contact with glass microspheres.

In a particular embodiment, the platelets are activated by a substance selected from calcium chloride, thrombin and collagen, preferably calcium chloride. Platelet activation is carried out by means of adding said substance to the plasma obtained after adjusting the pH. In a more preferred embodiment, platelet activation is carried out by means of adding a calcium chloride solution, preferably an aqueous calcium chloride solution of at least 5%, such as 5 to 30%, more preferably 10% by weight of calcium chloride relative to the volume of the solution (w/v in g/100 mL). The calcium chloride solution, in any of the mentioned concentrations, is preferably added in a volume of between 0.05% and 1%, more preferably in a volume of about 0.2%, with respect to the volume of the plasma obtained after adjusting the pH.

In a particular embodiment, platelet activation is performed by means of adding a CaCl₂ solution, as described above, at a concentration of between 20 µl/ml of plasma and 60 µl/ml of plasma, between 30 µl/ml of plasma and 50 µl/ml of plasma, more particularly of about 40 µl/ml of plasma.

In a particular embodiment, after obtaining the red blood cell-free plasma or red blood cell- and white blood cell-free plasma, but before performing concentration, the platelet and total protein concentration in said red blood cell-free plasma or red blood cell- and white blood cell-free plasma is measured to check that they remain at values similar to those of the anticoagulated blood from which said plasma is obtained. Similar value is understood to mean values which are equal to those of the anticoagulated blood from which said plasma is obtained or which differ by up to 1%, 2%, 3%, 4%, 5%, 6%, 7%, 8%, 9% or up to 10% with respect to the values of the anticoagulated blood from which said plasma is obtained.

In order to obtain the concentration factors of PRP proteins and platelets in relation to the levels in blood, respective analyses of blood samples and their corresponding PRP samples were performed after processing according to the invention. Platelet concentration can be measured by means of a hematology analyzer based on fluorescence flow cytometry technology (Sysmex XS-1000i; Kobe, Japan). The samples were measured using the "Complete Blood Count with Differential test". Total protein concentration can be measured with a Cobas c 501 analyzer (Roche, Basel, Switzerland).

In another particular embodiment, once the concentrated plasma has been obtained after the evaporation process, the platelet and total protein concentration of said concentrated plasma is measured. Platelet and total protein concentration is measured as indicated above. The objective of said measurement is to check that the platelet and total protein concentration has increased with respect to the platelet concentration measured in the red blood cell-free plasma or the red blood cell- and white blood cell-free plasma prior to concentration or with respect to the anticoagulated blood from which said plasma is obtained.

In another embodiment, platelets are activated mechanically by means of contact with glass microspheres.

In one embodiment, the method of the invention does not comprises any lyophilization step.

### Enriched plasma

In a second aspect, the invention relates to a plasma enriched in platelets and plasma molecules obtained by means of the method of the first aspect of the invention.

In a particular embodiment, the platelet and total protein concentration in the enriched plasma is at least 1.5 times higher than that of the anticoagulated blood sample of a subject in step i) of the method of the invention.

The plasma obtained by means of the method of the invention contains a higher platelet concentration than that of the anticoagulated blood of a subject in step i) of the method of the invention. In a particular embodiment, the plasma obtained by means of the method of the invention contains a platelet concentration of between 1.2 and 5 times higher than that of the anticoagulated blood of a subject in step i) of the method of the invention. In another particular embodiment, the plasma obtained by means of the method of the invention contains a platelet concentration of between 1.5 and 3, between 1.6 and 2 times higher than that of the levels in the anticoagulated blood of a subject in step i) of the method of the invention.

In a particular embodiment, the platelet concentration in the enriched plasma is of between 200,000 and 600,000 platelets per microliter of plasma (pl/µl), between 225,000 and 550,000 platelets per microliter of plasma (pl/µl), between 250,000 and 500,000 platelets per microliter of plasma (pl/µl), between 300,000 and 450,000 platelets per microliter of plasma (pl/µl), more preferably between 225,000 and 525,000 platelets per microliter of plasma (pl/µl).

The plasma obtained by means of the method of the invention contains a total protein concentration higher than that of the anticoagulated blood of a subject in step i) of the method of the invention. In a particular embodiment, the total protein concentration of the plasma obtained by means of the method of the invention is of between 1.2 and 5 times higher than that of the anticoagulated blood of a subject in step i) of the method of the invention, between 1.5 and 3 times higher than that of the anticoagulated blood of a subject in step i) of the method of the invention, between 1.7 and 2 times that of the anticoagulated blood of a subject in step i) of the method of the invention.

The amount of total protein may vary according to the subject, but in general the range of total protein varies between 6 and 8.3 g/mL.

In a preferred embodiment, the plasma obtained by means of the method of the invention contains:
- a platelet concentration of between 1.2 and 5, particularly between 1.5 and 3, more particularly between 1.6 and 2 times that of the anticoagulated blood sample of step i); and
- a total protein concentration of between 1.2 and 5, particularly between 1.5 and 3, more particularly between 1.7 and 2 times that of the anticoagulated blood sample of step i).

In one embodiment, the proportion between platelet concentration and total protein concentration (with total protein concentration being representative of plasma protein concentration) in the plasma obtained by means of the method of the invention maintains the equilibrium of the anticoagulated blood sample of step i), i.e., the proportion between platelet concentration and total protein concentration in the plasma obtained by means of the method of the invention differs from the same proportion in the anticoagulated blood sample of step i) by no more than 20%, preferably no more than 10%, more preferably no more than 5%.

The plasma obtained by means of the method of the invention contains an albumin concentration higher than that of the anticoagulated blood of a subject in step i). More specifically, the plasma obtained by means of the method of the invention contains an albumin concentration of between 1.2 and 5 times higher than that of the anticoagulated blood of a subject in step i) of the method of the invention, between 1.5 and 3 times higher than that of the anticoagulated blood of a subject in step i) of the method of the invention, between 1.7 and 2 times higher than that of the anticoagulated blood of a subject in step i) of the method of the invention.

The plasma obtained by means of the method of the invention has an IGF-I plasma factor level higher than that of the anticoagulated blood of a subject in step i) of the method of the invention. In another particular embodiment, the plasma obtained by means of the method of the invention has an IGF-I plasma factor level of at least between 1.2 and 5 times higher, of at least between 1.5 and 3 times higher than that of the anticoagulated blood of a subject in step i) of the method of the invention, between 1.8 and 2.2 times higher than that of the anticoagulated blood of a subject in step i) of the method of the invention.

The range of IGF-1 in blood varies according to the age of the subject. Taking into account all ages, the range of IGF-1 in blood varies between 30 and 640 ng/mL.

In the context of the present invention, the expression "higher" always refers to a concentration of the compounds being analyzed that is higher than that of the blood of a control subject, which corresponds to the anticoagulated blood of the same subject in step i) of the method of the invention. The different methods for preparing PRPs have an impact on the capacity to preserve and concentrate the different components of the anticoagulated blood. In this sense, it has been observed that the plasma obtained according to the present invention has surprisingly high levels of certain growth factors, such as IGF-I.

The term IGF-I refers to insulin-like growth factor 1, also known as somatomedin C or mechano growth factor (MGF). In a particular embodiment, IGF-I is the protein encoded by the human gene shown in the Ensembl database under accession number ENSG00000017427 (Ensembl release 106, April 2022). The protein encoded by this gene is similar to insulin in function and structure and is a member of a family of proteins involved in mediating growth and development. The encoded protein is processed from a precursor, binds to a specific receptor and is secreted.

The above total protein measurements are performed before platelet activation and degranulation. Therefore, the above values refer only to the contents of plasma proteins and not to the contents of plasma proteins plus platelets.

The concentration of specific platelet or plasma molecules, such as IGF-1, HGF, and PDGF-AB, can be analyzed as follows. First, plasma samples are activated by adding a 10% aqueous CaCl₂ solution in a proportion of 40 µL per 1 mL of plasma. Then, the concentration of those molecules is measured by means of a commercially available enzyme-linked immunosorbent assay (ELISA) kit following the manufacturer's instructions (R&D Systems, Minneapolis, USA).

### Medical uses

In a third aspect, the invention relates to a plasma enriched in platelets and plasma molecules according to the second aspect of the invention (hereinafter "plasma of the invention") for use in medicine.

As mentioned above, the plasma obtained by means of the method of the present invention has concentrated levels of both platelets (and therefore platelet growth factors) and non-platelet biomolecules, particularly non-platelet growth factors, among which the high IGF-I content, having properties related to cell growth, proliferation and differentiation of mesenchymal cells that create connective tissues (muscle, blood vessels, cartilage, bone, etc.), are highlighted.

Higher platelet concentration improves the healing properties of the plasma with respect to plasmas in which platelets are not concentrated, such as plasma isolated directly from blood.

In relation to the plasma of the invention, the inventors have surprisingly discovered that by also concentrating non-platelet biomolecules, particularly growth factors such as IGF-I, by means of the method of the invention, the regenerative potential of the plasma is enhanced.

Therefore, in another further aspect, the present invention relates to a plasma enriched in platelets and plasma molecules according to the second aspect of the invention for use in regenerative medicine.

More particularly, use in regenerative medicine refers to the treatment of injured tissue in a subject.

Likewise, the present invention relates to a method for the treatment of injured tissue in a subject in need thereof, which comprises administering the plasma of the invention to the subject.

Likewise, the present invention relates to the use of the plasma of the present invention in the preparation of a medicinal product for the treatment of injured tissues.

As used herein, the term "treating" (or "treat" or "treatment") refers to processes which involve slowing, interrupting, controlling, stopping, reducing or reversing the progression or severity of an existing symptom, disorder, condition or disease, but does not necessarily involve the complete elimination of all symptoms, conditions or disorders related to the disease. Treatment of a disorder or disease may, for example, stop the progression of the disorder or disease (e.g., without deterioration of symptoms) or delay the progression of the disorder or disease (in case the interruption of progression is only transient in nature). "Treatment" of a disorder or disease may also lead to a partial response (e.g., improvement of symptoms) or a complete response (e.g., disappearance of symptoms) of the subject/patient suffering from the disorder or disease. Accordingly, "treatment" of a disorder or disease may also refer to an improvement of the disorder or disease which may, for example, stop the progression of the disorder or disease or delay the progression of the disorder or disease. Such partial or complete response may be followed by a relapse. It should be understood that a subject/patient may experience a wide range of responses to a treatment.

As used herein, the term "injured" is used in its ordinary sense to refer to any tissue damage, but preferably refers to a wound, trauma or ulcer.

In a preferred embodiment, the injured tissue is musculoskeletal tissue.

In a particular embodiment, the injured tissue is bone or cartilage.

In another particular embodiment, the injured tissue is a soft tissue. Soft tissue refers to all tissue in the body that is not hardened by ossification or calcification processes such as in bones and teeth. In a more particular embodiment, the injured tissue is selected from the group consisting of connective tissue, cardiac muscle, skeletal muscle, brain tissue, corneal tissue, nervous tissue and vascular tissue.

Examples of specific pathological conditions that can be treated with the plasma of the present invention are articular cartilage injury, skeletal muscle injury, ligament injury, osteoporosis, age-related wasting or muscular dystrophy.

In another particular embodiment, the plasma of the present invention is used in odontology, particularly after oral surgery.

The plasma enriched in platelets and plasma molecules can function as a scaffold and be used as an implant *per se,* to provide mechanical support to a defective or injured site *in situ* and/or to provide a matrix within which cells at the defective or injured site can proliferate and differentiate.

The plasma of the invention can be administered in any suitable dose. In some embodiments, the dose may be between 1 ml and 20 ml. The dose is generally determined according to the specific medical procedure followed, the treated condition and the patient profile.

The plasma of the invention can be administered orally and parenterally, such as intravenously (IV), intraperitoneally (IP), subcutaneously (SE), intramuscularly (IM), rectally, topically, ophthalmically, nasally and transdermally. The plasma of the invention can be administered to a subject in need thereof by means of injection using a syringe or catheter. The plasma of the invention can also be administered through a dermal patch, a spray device, or in combination with an ointment or bone graft. It can also be used as a coating on sutures, stents, screws, plates, or any other implantable medical device. The plasma of the invention formulated as gels or other viscous liquids may be difficult to administer through a needle or syringe. Therefore, in variations in which the use of a needle or syringe is desired, it may be desirable to add a gelling and/or hardening agent to the plasma of the invention *in situ.*

The site of administration of the PRP composition is typically the site of tissue damage or its proximity. The site of tissue damage is determined by means of well-established methods including imaging studies and patient feedback or a combination thereof. In some examples, the plasma of the invention can be administered to the damaged connective tissue in or around the affected joints.

Likewise, platelets play a central regulatory role in all phases of inflammation and subsequent healing by means of releasing all these factors from their α-granules following stimulation. In contrast to granular α agents, mediators released from dense bodies exert effects that are more restricted to the initial phase of inflammation. On the other hand, IGF-I, which is concentrated particularly in the plasma of the invention, has proven to be very useful in inflammatory pathologies, such as osteoarthritis (Wen C et al., 2021, Arthritis Research and Therapy, col. 23, 277, and Mullen LM et al., 2015, Journal of materials science. Materials in Medicine, vol. 26,1 (2015): 5325).

Therefore, in another aspect, the invention relates to the plasma of the invention for use in the treatment of inflammatory pathologies.

Examples of specific inflammatory pathologies which can be treated with the plasma of the present invention are osteoarthritis, arthritis, tendonitis, fibrosis, bursitis, among others.

Likewise, the present invention relates to a method for the treatment of inflammatory pathologies in a subject in need thereof, which comprises administering the plasma of the invention to the subject.

Likewise, the present invention relates to the use of the plasma of the present invention in the preparation of a medicinal product for the treatment of inflammatory pathologies.

In a particular embodiment, the plasma subjected to the method of the present invention is administered to the same patient providing the anticoagulated blood sample of a subject in step i) from which said enriched plasma was obtained. In other words, in a particular embodiment, the plasma subjected to the method of the present invention and then administered to the subject is autologous.

In a particular embodiment, the plasma subjected to the method of the present invention is administered to a different patient from the patient providing the anticoagulated blood sample of a subject in step i) from which said enriched plasma was obtained. In other words, in a particular embodiment, the plasma subjected to the method of the present invention and then administered to the subject originates from a donor, i.e., it is allogenic.

In another aspect, the invention relates to the cosmetic use of a plasma obtained by means of the method of the present invention. Particular cosmetic uses are the treatment of wrinkles on skin, stretch marks or eye bags.

The plasma of the present invention can be added to a wide variety of products for cosmetic application, including make-up, creams for cleansing, protecting, treating or caring for skin, particularly the face, hands and feet (for example, day and night creams, make-up remover creams, foundation creams and sunscreens), liquid foundations, make-up remover lotions, skin care or protective body lotions, sun protection lotions, skin care lotions, gels or foams, such as cleansers, sunscreens and artificial tanning lotions, bath preparations, deodorant compositions, aftershave gels or lotions, depilatory creams and compositions for insect bites and pain. The plasma of the invention may adopt a wide variety of forms and include, for example, dressings, lotions, solutions, aerosols, creams, gels, ointments or the like.

The treatment method described herein is advantageous because it requires minimal preparation for use by the physician.

The invention will be described through the following examples which should be considered merely illustrative and non-limiting with respect to the scope of the invention.

### EXAMPLES

### Technical description of the process

The following process has been followed to develop this product:
1. Blood was drawn from the patient using extraction tubes having a volume of 9 mL with 3.8% sodium citrate as an anticoagulant. The number of tubes depends on the volume of product to be obtained. For example, to obtain 6 mL of concentrated plasma, 12 mL of initial plasma will be needed, and to obtain 12 mL of plasma, 24 mL of blood (3 tubes) will be needed.
2. The tubes containing blood were centrifuged at 1200xg for 8 minutes.
3. The entire plasma fraction was collected without including white blood cells and red blood cells.
4. Platelet concentration both in blood and in plasma was measured to check that they remained at similar values.
5. The plasma was introduced into a container (flask) to proceed with evaporation using the rotary evaporator. The temperature of the bath (water-filled container) of the rotary evaporator was adjusted to 37°C and the flask was introduced therein, rotating it so that the sample was distributed over the entire surface and thus achieving faster evaporation of the sample. Finally, in order to be able to proceed with evaporation, a vacuum of -920 mbar was applied. After 3-5 minutes, the sample was concentrated to half its initial volume (from 12 mL to 6 mL). A smaller or larger final volume can be obtained at a higher or lower concentration by increasing or decreasing the process time.
6. The plasma sample concentrated using a rotary evaporator (PC-RV) was collected and the number of platelets was measured to calculate their concentration with respect to the baseline value and to confirm that the platelet concentration has doubled. Furthermore, it was observed that when the water in the plasma evaporated, the plasma was basified to a pH of 8.6, compared to physiological pH (7.4), presumably due to the ions existing in the plasma such as sodium, calcium, magnesium, chlorine, phosphorus and potassium, among others, also being concentrated.
7. The pH of the concentrated plasma was adjusted to pH 7.4 by adding a 0.8 M hydrochloric acid (HCI) buffer at a percentage of 2.3% (any other buffer that acidifies the sample can be used). It was found that pH adjustment is essential for the occurrence of the desired coagulation resulting from platelet activation prior to treatment, and therefore for obtaining greater cell proliferation. The amount of CaCl₂ used for the activation of the coagulation cascade was 40 µL/1 mL of PC-RV.

### Technical analysis of the product obtained

In order to be able to verify that the concentrated plasma according to the invention (referred to as PC-RV) has tissue regeneration properties, different experiments were performed to see if an improvement in the treatment is achieved with respect to conventional PRPs. Specifically, a comparison has been made with:
1. A conventional/standard PRP referred to as sPRP which is obtained by means of centrifugation and which doubles the platelet concentration with respect to blood but in which the plasma protein (molecule) concentration is the same as that of blood.
2. A PRP enriched with proteins and plasma molecules of the state of the art, specifically, it corresponds to that disclosed in document WO2018206756A1, which has been referred to herein as PRP-MT.

The experiments performed are described below.

### 1. PC-RV vs. sPRP comparative data

### Platelet, total protein and albumin concentration

It has been verified that by concentrating the plasma sample, platelets, total proteins and albumin have been concentrated about twice as much with respect to baseline levels in blood.

Platelet concentrate is important to be able to confirm that a platelet-rich plasma is obtained. Moreover, total protein and albumin concentration indicates whether the plasma growth factors of interest have been able to be concentrated to twice as much.

The relationship of platelet, total protein and albumin concentration in concentrated plasmas with respect to baseline levels in blood can be seen in Figure 1.

### Ion concentration

It has been observed that having a high salt/ion concentration in the plasma sample causes the pH to increase and this prevents the start of the coagulation cascade, unless the pH is adjusted and twice the concentration of CaCl₂ is added. The relationship of the levels of ions existing in PC-RV with respect to sPRP can be seen Figure 2.

### Growth factor concentration (ELISAs)

Total protein analysis has allowed verifying that it is possible to concentrate the proteins present in the plasma to twice as much. To verify the concentration of some of the proteins of interest specifically, analysis has been performed by means of using the ELISA technique.

The relationship of the concentration of the different growth factors with respect to values in sPRP after platelet activation by means of CaCl₂ can be seen in Figure 3.

By observing the results obtained, it is concluded that the new PRP, PC-RV, shows differences in the levels of plasma factors with respect to sPRP.

### Cell viability

In order to see whether PRP enriched in plasma factors has a greater response in cell proliferation with respect to conventional/standard PRP (sPRP), cell media were supplemented with 10% of the serum originating from the different formulations, after activation with CaCl₂. A growth curve of dermal fibroblasts cultured *in vitro* with media supplemented with different sera, sPRP or PC-RV can be seen in Figure 4. Measurements were performed every 24 h for a total of 72 h. A luminescence-based cell viability measurement method was used to that end.

Based on the results, it can be concluded with statistical significance that PC-RV promotes greater cell growth than sPRP on dermal fibroblasts. This experiment was performed on 5 different donors, obtaining similar results in all of them. Even in individuals who were poor responders, it was observed that concentrated sera showed an improvement in terms of proliferation (Figure 4).

By way of result validation, immunohistochemical staining was performed to evaluate cell density under different conditions. Several images in which dermal fibroblasts have been stained after being cultured in media with different sera for 72 h can be seen in Figure 5. Staining was performed with phalloidin (cytoskeletal stain) and Hoechst (nuclear stain). A higher cell density in cells cultured with PC-RV with respect to the negative control of media without serum and sPRP can be seen in the images.

### 2. PC-RV vs. PRP-MT comparative data

### Platelet, total protein and albumin concentration

PC-RV is a platelet-rich plasma (PRP) with a mean platelet concentration of 1.7 times (range 1.25 - 2.53) with respect to levels in blood (Figure 6). If these results are compared with those obtained by the closest device in the state of the art, which corresponds to a plasma enriched with platelets and plasma molecules disclosed in patent application WO2018/206756A1 (WO'756) and the plasma of which is referred to as PRP-MT, similar platelet levels are found, with no statistically significant differences between both systems.

Moreover, total protein and albumin levels, indicative of the concentration of plasma growth factors of interest, have been measured, although this will be confirmed by means of specific ELISA tests, which are shown below.

The relationship of total protein and albumin concentration in PC-RV with respect to baseline levels in blood can be seen in Figure 7. In this case, the document (WO'756) does not provide data describing this aspect.

### Ion concentration

The relationship of the levels of ions existing in PC-RV with respect to blood can be seen in Figure 8. Therefore, it has been seen that salt level increases after rotary evaporation. However, this problem is solved throughout sample processing, since the pH of the plasma (pH 8.4) subjected to rotary evaporation is corrected by means of an acid buffer, returning it to the baseline pH of the plasma (pH 7.4). In this case, 0.8 M HCI was used, but any other that buffers the sample in the same range could be used. Furthermore, the addition of 20% of CaCl₂ allows triggering the coagulation process.

### Growth factor concentration (ELISAs)

Total protein analysis has allowed verifying that it is possible to concentrate the proteins present in the plasma twice as much. To verify the concentration of some of the proteins of interest specifically, analysis of different growth factors has been performed by means of the ELISA technique. Moreover, since PC-RV is a PRP, i.e., a formulation that concentrates the platelets to twice as much, it was desired to verify that the platelet factors doubled in comparison with baseline levels. To that end, the measurement of the platelet factor PDGF-AB was chosen. This increased proportionally to the platelet concentration level.

Following this confirmation, two plasma factors (IGF-I and HGF) were measured to confirm that they are concentrated by means of this processing, unlike conventional PRPs, where only platelet factors are concentrated.

The relationship of the growth factor concentration in the different systems (PRP-MT and PC-RV) relative to baseline levels can be seen in Figure 9. IGF-I was concentrated in both systems proportionally to the volume of water removed. However, in PC-RV this increase in concentration was statistically higher. In the case of HGF, the results showed significant differences between the two systems, since in WO'756 they were concentrated in proportion to the volume of water removed and, however, in PC-RV there was no concentration of this factor.

These results confirm that PRP-MT and PC-RV are two products with different compositions, which could generate differences in their bioactivity, which was verified by means of an *in vitro* study where their impact on cell proliferation was measured.

### Cell viability

In order to analyze whether PC-RV has a greater response in cell proliferation with respect to PRP-MT, the cell media was supplemented with 10% of the serum originating from the different formulations, after their activation with CaCl₂. The growth ratio of dermal fibroblasts cultured in *vitro* with media supplemented with PRP-MT and PC-RV can be seen in the following graph. Measurements were performed at 0, 48 and 72 h. Therefore, the results were expressed in cell proliferation ratios, i.e., cell density at 48 or 96 h with respect to time zero. Based on the results, it can be concluded that PC-RV clearly promotes greater cell growth than WO'756 on dermal fibroblasts (Figure 10).

By way of validation of these results, immunofluorescence staining was performed to evaluate cell density under the different conditions. Several images in which dermal fibroblasts have been stained after being cultured in media with the different sera for 72 h can be seen in Figure 11. Staining was performed with phalloidin (cytoskeletal stain) and Hoechst (nuclear stain). An increase in cell density in cells cultured with PC-RV after 72 h (B) with respect to the beginning (A) can be seen in the images.

## Claims

1. An *in vitro* method for producing a plasma enriched in platelets and plasma molecules which comprises:
i) obtaining a red blood cell-free plasma from an anticoagulated blood sample of a subject, wherein the plasma comprises platelets and plasma molecules;
ii) concentrating the platelets and plasma molecules present in the plasma obtained in step i) by means of evaporation;
iii) adjusting the pH of the concentrated plasma obtained in step ii) to a value of between 6 and 7.6, preferably between 6.5 and 7.5; more preferably to a pH of 7.4.

2. The method according to claim 1, wherein in step i) a red blood cell- and white blood cell-free plasma is obtained from anticoagulated blood of a subject.

3. The method according to any of claims 1 or 2, wherein evaporation is performed by rotary evaporation.

4. The method according to any of claims 1 to 3, wherein during evaporation the plasma is subjected to a temperature of between 30°C and 45°C, preferably between 33°C and 40°C, more preferably 37°C.

5. The method according to any of claims 1 to 4, wherein during evaporation the plasma is subjected to an applied vacuum of between 250 and 1000 mbar, preferably between 500 and 1000 mbar, more preferably 920 mbar.

6. The method according to any of claims 1 to 5, wherein evaporation is performed for a period of between 1 minute and 30 minutes.

7. The method according to any of claims 1 and 3 to 6, wherein the red blood cell-free plasma is obtained by means of centrifuging the anticoagulated blood sample at a speed of between 700 and 1500 g for between 5 and 15 minutes and collecting the supernatant and the buffy coat from the centrifuged blood sample.

8. The method according to any of claims 2 to 6, wherein the red blood cell- and white blood cell-free plasma is obtained by means of centrifuging the anticoagulated blood sample at a speed of between 700 and 1500 g for between 5 and 15 minutes, and collecting the supernatant from the centrifuged blood sample.

9. The method according to any of claims 1 to 8, further comprising the step of activating the platelets obtained in the concentrated plasma obtained after adjusting the pH.

10. The method according to claim 9, wherein platelet activation is performed by means of adding a compound selected from calcium chloride, thrombin and collagen.

11. The method according to claim 10, wherein platelet activation is performed by means of adding calcium chloride.

12. The method according to any of claims 1 to 11, wherein the concentration of platelets and total proteins in the enriched plasma is at least 1.5 times higher than that of the anticoagulated blood sample of a subject in step i).

13. The method according to any of claims 1 to 12, wherein the concentration of IGF-I is at least 1.5 times higher than that of the anticoagulated blood sample of a subject in step i).

14. The method according to any of claims 1 to 13, wherein the subject is a human being.

15. A plasma obtained by the method of any of claims 1 to 14.

16. The plasma defined in claim 15 for use in medicine.

17. The plasma for use according to claim 16 for use in regenerative medicine.

18. The plasma for use according to claim 17, wherein the plasma is used in the treatment of injured tissues.

19. The plasma for use according to claim 16 for use in the treatment of inflammatory pathologies.

20. The plasma for use according to any of claims 16 to 19, wherein the plasma is administered to the same patient providing the anticoagulated blood sample of a subject in step i) from which said plasma was obtained.

21. The plasma for use according to any of claims 16 to 19, wherein the plasma is administered to a patient different from the one providing the anticoagulated blood sample of a subject in step i) from which said plasma was obtained.

22. Cosmetic use of the plasma defined in claim 15.
